(19) Europäisches Patentamt

European Patent Office

Office européen des brevets

(11) Veröffentlichungsnummer: **0 326 063 B1**

(12) **EUROPÄISCHE PATENTSCHRIFT**

(45) Veröffentlichungstag der Patentschrift: **07.09.94**

(21) Anmeldenummer: **89101127.2**

(22) Anmeldetag: **23.01.89**

(51) Int. Cl.⁵: **C12P 13/00**

(54) **Enzymatisches Verfahren zur Herstellung von optisch aktiven Cyanhydrinen.**

(30) Priorität: **29.01.88 DE 3802624**
       **14.07.88 DE 3823864**

(43) Veröffentlichungstag der Anmeldung:
    **02.08.89 Patentblatt 89/31**

(45) Bekanntmachung des Hinweises auf die
   Patenterteilung:
   **07.09.94 Patentblatt 94/36**

(84) Benannte Vertragsstaaten:
   **AT BE CH DE ES FR GB IT LI NL**

(56) Entgegenhaltungen:

   **CHEMICAL ABSTRACTS, Band 107, 1987, Seite 346, Zusammenfassung Nr. 3690p, Columbus, Ohio, US; F. EFFENBERGER et al.: "Enzyme-catalyzed cyanohydrin preparation in organic solvents"**

   **BIOLOGICAL ABSTRACTS, Band 70, Nr. 7, 1980, Seite 4906, Philadelphia, US; J.M.SCHUMAN: "Studies on the kimetics of cyanohydrin synthesis and cleavage by the flavoenzyme oxynictrilase"**

(73) Patentinhaber: **FORSCHUNGSZENTRUM JÜLICH GMBH**

   **Wilhelm-Johnen-Strasse**
   **D-52425 Jülich (DE)**

   Patentinhaber: **Degussa Aktiengesellschaft**
   **Weissfrauenstrasse 9**
   **D-60311 Frankfurt (DE)**

(72) Erfinder: **Niedermeyer, Uwe**
   **Düffelstrasse 8**
   **D-5303 Walberberg (DE)**
   Erfinder: **Kragl, Udo**
   **Robert-Koch-Strasse 5**
   **D-5170 Jülich (DE)**
   Erfinder: **Kula, Maria-Regina, Prof.**
   **Selgenbusch 12**
   **D-5162 Hambach-Niederzier (DE)**
   Erfinder: **Wandrey, Christian, Prof.**
   **Wolfshovener Strasse 139**
   **D-5170 Jülich (DE)**
   Erfinder: **Makryaleas, Kyriakos, Dr.**
   **Hanauer Strasse 36**
   **D-6463 Freigericht 1 (DE)**
   Erfinder: **Drauz, Karlheinz, Dr.**
   **Zur Marienruhe 13**
   **D-6463 Freigericht 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Verfahren zur Herstellung von optisch aktiven Cyanhydrinen durch enzymatische Umsetzung von Oxoverbindungen mit Blausäure in Gegenwart von Oxynitrilase.

Optisch aktive Cyanhydrine, wie sie durch die Oxynitrilasekatalyse aus Aldehyden oder Ketonen entstehen, werden wie z. B. die Abkömmlinge des m-Phenoxybenzaldehyds und substituierter Analoga als Bausteine zur Synthese von photostabilen Pyrethroiden eingesetzt.

Weiter finden sie für die Synthese optisch aktiver α-Hydroxycarbonsäuren Verwendung.

Diese werden wiederum als Zusatzstoffe zu Futtermitteln oder zur Gewinnung pharmazeutischer Wirkstoffe, Vitamine und Flüssigkeitskristalle eingesetzt.

Diese optisch aktiven α-Hydroxycarbonsäuren lassen sich nach F. Effenberger et al., Angew. Chem. 95 (1983) Nr. 1, Seite 50, vorteilhaft in anderweitig nur sehr schwer herzustellende N-substituierte optisch aktive α-Aminosäuren überführen.

Optisch aktive Cyanhydrine geben ebenfalls einen leichten Zugang zu optisch aktiven α-Aminoalkoholen und weiteren daraus ableitbaren optisch aktiven Verbindungen, die in der Wirkstoffsynthese Bedeutung besitzen.

Aus der DE-PS 1 300 111 ist bereits ein Verfahren zur Herstellung von optisch aktiven (R)-Cyanhydrinen bekannt, bei dem Aldehyde in Gegenwart von Oxynitrilase mit Blausäure zur Reaktion gebracht werden. Bei diesem bekannten Verfahren erfolgt die Umsetzung in wäßrigem oder wäßrig-alkoholischem (50 % v/v) Reaktionsmilieu bei einem pH-Wert von 5,4 (bzw. 4,8 bis 5,4; siehe Becker u. a., JACS 1966 Seite 4299), d. h. dicht unterhalb des pH-Optimums der Enzymaktivität, das zwischen 5,6 und 6 liegt. Die optische Reinheit der nach diesem Verfahren erzeugten (R)-Cyanhydrine zeigte allerdings einige Mängel.

F. Effenberger u. a. (Angew. Chem. 99 (1987) S. 491-2) untersuchten die enzymatische Cyanhydrinbildung in wäßrig-alkoholischen Systemen unter Variation von pH-Wert, Temperatur und Konzentration im Hinblick auf eine optimale Unterdrückung der neben der enzymkatalysierten Addition einhergehenden, zu Racematen führenden chemischen Addition von Blausäure an die Aldehydgruppe. Da auf diese Weise keine befriedigende Optimierung gefunden werden konnte, schlagen F. Effenberger u. a. vor, die chemische Reaktion durch Verwendung organischer, mit Wasser nicht mischbarer Lösungsmittel zu unterdrücken. Insbesondere wurde mit Ethylacetat und unter Verwendung von trägerfixierter (R)-Oxynitrilase gearbeitet. Auf diese Weise wurden Produkte mit hoher optischer Reinheit erhalten, jedoch bedeutet die Umsetzung in rein organischem Milieu verfahrenstechnisch ein gewisses Hindernis, insbesondere, da Aktivität und Stabilität des Enzyms in diesem Milieu verringert sind.

Ziel der Erfindung ist es daher, ein Verfahren zur enzymatischen Cyanhydrinherstellung vorzuschlagen, bei dem im wäßrigen Milieu gearbeitet wird und trotzdem hohe optische Reinheiten erzielt werden können.

Das zu diesem Zweck entwickelte erfindungsgemäße Verfahren ist dadurch gekennzeichnet, daß man von aliphatischen, aromatischen oder heteroaromatischen Aldehyden oder Ketonen abgeleitete (R)-Cyanhydrine oder (S)-Cyanhydrine durch Umsetzung der entsprechenden Oxoverbindungen mit Blausäure in rein wäßrigem Milieu in Gegenwart von (R)-Oxynitrilase (4.1.2.10) bzw. Oxynitrilase (4.1.2.11) unter derart sauren Bedingungen und bei solchen Temperaturen herstellt, daß die chemische Konkurrenzreaktion und die Racemisierung gegenüber der enzymatischen Synthese vernachlässigbar sind.

Die aus Sorghum biocolor stammende Oxynitrilase (4.1.2.11) wurde z. B. von E. Bové et al. im J. Biol. Chem. 236 (1961) 207 beschrieben. Ihre Brauchbarkeit für die Gewinnung von optisch reinen (S)-Cyanhydrinen war jedoch bisher unbekannt. Sie wird im nachfolgenden auch einfach als (S)-Oxynitrilase

Es wurde überraschenderweise gefunden, daß die Aktivität der Enzyme im Hinblick auf die Cyanhydrinsynthese bei relative niedrigen pH-Werten zwar vermindert aber noch ausreichend ist, bei denen die chemische Addition von Cyanwasserstoff und auch die Racemisierungsreaktionen des gebildeten Produkts unterdrückt werden können. Der jeweils optimale pH-Bereich hängt dabei in gewissem Umfang von den eingesetzten Substraten ab, wobei allgemein pH-Werte bis maximal etwa 4,5 bevorzugt werden.

Dabei wird in rein wäßrigem Milieu , d.h. ohne Zusatz von organischen Lösungsmitteln, gearbeitet, um den enzymaktivitätsmindernden Einfluß der Anwesenheit auch nur geringer Mengen an organischen Lösungsmitteln wie z.B. Ethanol zu vermeiden.

Die chemische Reaktivität der Carbonylverbindung sowie deren Affinität zum Enzym bestimmen, wie tief der pH-Wert in Abhängigkeit von der Temperatur zu senken ist, damit die chemische Cyanhydrinsynthese neben der enzymatischen zu vernachlässigen ist. pH-Werte unter 2,8 sind allerdings nicht zu empfehlen, da die Oxynitrilasen dann zu rasch desaktiviert werden.

Die Arbeit in wäßrigem Milieu ermöglicht den Einsatz des jeweiligen Enzyms in gelöster Form und erleichtert die Dosierung erheblich. Ferner ist so eine kontinuierliche Arbeitsweise, insbesondere im Enzymmenbranreaktor einfach durchführbar und die Aufarbeitung durch Extraktion der gebildeten Cyanhydrine mit wasserunlöslichen bzw. mit Wasser nicht mischbaren Lösungsmitteln, wie z. B., Methylenchlorid, Chloroform, 1,2-Dichlorethan, Essigsäureethylester, Essigsäureisopropylester, Methyl-tert.butyl-ether, ohne weiteres möglich.

In Kauf genommen wird bei der erfindungsgemäßen Reaktion, daß die enzymatische Aktivität und die Haltbarkeit des Enzyms mit abnehmendem pH-Wert schlechter werden, jedoch sind die (R)-Oxynitrilase (4.1.2.10) aus Prunus amygdalus und die Oxynitrilase (4.1.2.11) aus Sorghum bicolor ohne weiteres zugänglich und käuflich zu erwerben, so daß der Einsatz erhöhter Mengen und eine evtl. notwendige Nachlieferung ohne weiteres möglich sind, insbesondere, da die Minderung der Enzymstabilität nicht übermäßig hoch ist (z. B. 8 % pro Tag bei pH 3,75 und 20°C für (R)-Oxynitrilase).

Gemäß der Erfindung können alle Carbonylverbindungen, die Substrate für die Oxynitrilasen sind und bei denen sich die rein chemische Cyanhydrinbildung durch eine Absenkung des pH-Wertes bis auf 2,8 unterdrücken läßt, unter Erzielung des (R)- bzw. (S)-Cyanhydrins mit höchster optischer Reinheit (ee > 99 %) umgesetzt werden.

Neben der Bildung aliphatischer Cyanhydrine wurde die Umsetzung von aromatischen Aldehyden besonders untersucht, insbesondere die Herstellung von (R)- bzw. (S)-Mandelsäurenitril und deren Derivaten ausgehend von ggf. substituiertem Benzaldehyd, wobei mit pH-Werten von 3,25 eine optische Reinheit von über 99 % ee erhalten wurde. Auch mit pH-Werten von 3,75 ist die chemische Synthese noch so weit unterdrückt, daß unter sonst identischen Bedingungen eine optische Reinheit über 98 % ee erhalten wird.

Carbonylverbindungen, die weniger reaktiv als Benzaldehyd sind, können entsprechend bei höheren pH-Werten ohne Einbuße an Enantioselektivität umgesetzt werden: so kann Furfurol unter sonst identischen Bedingungen, jedoch bei pH 4,0 zum (R)-Cyanhydrin mit einer optischen Reinheit von 99 % ee umgesetzt werden.

Durch Variation der substratkonzentration in einem weiteren Bereich und/oder Absenkung der Reaktionstemperatur ist es zusätzlich möglich, die chemische Begleitreaktion zu hemmen, so daß ein gewisser Spielraum für möglich günstigste Bedingungen gegeben ist.

Gemäß der Erfindung können insbesondere als Substrate aliphatische, aromatische und heterocyclische Aldehyde oder Ketone in Gegenwart von (R)-Oxynitrilase bzw. aromatische oder heteroaromatische Aldehyde oder auch Ketone mit (S)-Oxynitrilase umgesetzt werden, unter Bildung der optisch reinen (R)- bzw. (S)-Enantiomeren der entstehenden Cyanhydrine. Ein besonderes Interesse aus dieser Gruppe galt den aromatischen Aldehyden im Hinblick auf die Verwendung der gebildeten Cyanhydrine im pharmazeutischen Bereich.

Nachfolgend wird die Erfindung anhand von Beispielen näher verständlich werden, wobei in den Beispielen 1 bis 13 die Herstellung von (R)-Enantiomeren und in den Beispielen 14 bis 18 die Bildung von (S)-Enantiomeren beschrieben wird.

Beispiel 1:

53 mg (0,5 mmol)Benzaldehyd wurden in 9,4 ml 50 mM Citratpuffer von pH 3,25 gelöst und auf 20°C temperiert. Hierzu wurden 125 μl wäßrige (R)-Oxynitrilase-Lösung (ca. 0,24 mg) und 475 μl 4,2 M wäßriger HCN-Lösung hinzugegeben. Die Reaktion wurde polarimetrisch (λ = 578 nm) verfolgt. Nach 30 bis 45 Min. stellte sich ein konstanter Drehwert ein, und die Reaktion war beendet. Danach wurde das Reaktionsgemisch viermal mit 10 ml Chloroform extrahiert.

Die vereinigten organischen Phasen wurden über Natriumsulfat getrocknet und das Lösungsmittel am Rotationsverdampfer abgezogen. Anschließend wurde der Rückstand dreimal mit je 10 ml Pentan gewaschen und das Produkt im Vakuum getrocknet.

| Chem. Ausbeute | 61,8 mg (93 % d. Th.) |
| Optische Reinheit | > 99 % ee |

Die Bestimmung der optischen Reinheit erfolgte als N,O-Bis-(Pentafluorpropionyl)-2-amino-1-phenylethanol-Derivat des (R)-Mandelsäurenitrils kapillargaschromatographisch nach H. Frank et al. (J. Chromatogr. 146, (1987), 197-206) auf einer chiralen Trennphase (FS-Chirasil-Val, 25 m * 0,32 mm).

Die Derivatisierung wurde wie folgt vorgenommen: 1 - 2 mg des Mandelsäurenitrils wurden mit 250 μl einer 1 M Diboranlösung (in Tetrahydrofuran) in Chloroform bei Zimmertemperatur in 30 Min. reduziert. Nach Hydrolyse des überschüssigen Diborans mit einigen Tropfen Ethanol und Abziehen des Lösungsmittels wurde der erhaltene Aminoalkohol direkt mit 20 μl Pentafluorpropionsäureanhydrid in Methylenchlorid bei Zimmertemperatur in 15 Min acyliert. Schließlich wurde überschüssiges Anhydrid am Rotationsverdampfer abgezogen, der Rückstand mit Methylenchlorid wieder aufgenommen und gaschromatographisch analysiert.

Beispiel 2:

Entsprechend Beispiel 1 wurden 48 mg (0,5 mmol) Furfurol bei pH 4,0 umgesetzt. Nach ca. 30 Minuten war die Reaktion beendet. Die Isolierung des (R)-Cyanhydrins erfolgte wie in Beispiel 1 beschrieben.

| Chem. Ausbeute | 55,3 mg (90 % d. Th.) |
| Optische Reinheit | 99 % ee |

Zur Bestimmung der optischen Reinheit wurde das Cyanhydrin mit (R)-α-Methoxy-α-trifluormethylphenylessigsäurechlorid nach D. Elliot, V. M. D. Choi, W. S. Johnson (J. Org. Chem. 48 (1983), 2294-2295) in die diastereomeren Ester überführt und das Diastereomerenverhältnis kapillargaschromatographisch (FS-OV 1 10 m * 0,32 mm) ermittelt.

Beispiel 3:

Entsprechend Beispeil 1 wurden 22 mg (0,5 mmol) Acetaldehyd bei pH 3,25 umgesetzt. Nach ca. 90 Min. war die Reaktion beendet. Die Aufarbeitung erfolgte analog zu Beispiel 1.

| Chem. Ausbeute | 26 mg (73 % d. Th.) |
| Optische Reinheit | 76 % ee |

Die Bestimmung der optischen Reinheit erfolgte über das N,O-Bis-Pentafluorpropionyl-1-amino-2-propanol gemäß Beispiel 1.

Beispiel 4:

Entsprechend Beispiel 1 wurden 56 mg (0,5 mmol) 3-Methylcyclohexanon bei pH 4,0 umgesetzt. Die Reaktion war nach 90 Min. beendet.

| Chem. Ausbeute | 57 mg (83 % d. Th.) |
| Optische Reinheit | nicht bestimmt. |

Beispiel 5:

Kontinuierliche Produktion von (R)-Mandelsäurenitril im Enzym-Membran-Reaktor (EMR).
Im EMR wurde das Verfahren kontinuierlich mit erhöhten Mengen durchgeführt. Die Betriebsbedingungen des EMR (entspricht kontinuierlich betriebenem Rührkesselreaktor) gestatten die Verwendung relativ hoher pH-Werte, entsprechend milden Bedingungen, ohne daß ein Verlust an optischer Reinheit des Produktes auftritt. Die kontinuierliche Produktion führt zu einer ökonomischeren Ausnutzung des Enzyms, da es vor der Produktaufarbeitung durch Ultrafiltration abgetrennt wird.
In kontinuierlicher Arbeitsweise wurden so in einem 10 ml EMR in 26 Stunden 7 g optisch reines (R)-Mandelsäurenitril (ee > 99 %) produziert.

EP 0 326 063 B1

| Betriebsbedingungen: | | |
|---|---|---|
| Benzaldehyd: | 48 mmol/l | |
| HCN: | 180 mmol/l | |
| Citratpuffer: | 45 mmol/l | ph 3,75 |
| Verweilzeit: | 10 min | |
| Enzymkonzentration: | 0.38 mg/ml | |
| mittlerer Umsatz: | 84 % | |
| Raumzeitausbeute: | 773 g/(l • d) | |
| Temperatur: | 20°C | |

Beispiel 6

Entsprechend Beispiel 1 wurde eine 0,2 molare Isobutyraldehydlösung bei pH = 3,6 und T = 6°C umgesetzt. Die Reaktion war nach 90 Minuten beendet.

| Chemische Ausbeute: | 84 % d. Th. |
|---|---|
| Optische Reinheit: | 96 % ee |
| | $[\alpha]_D^{20}$ = +16,3° (c = 5 in $CHCl_3$) |

Beispiel 7

Entsprechend Beispiel 1 wurde eine 0,25 molare Isovaleraldehydlösung bei pH = 3,7 und T = 7°C umgesetzt. Die Reaktion war nach 100 Minuten beendet.

| Chemische Ausbeute: | 92 % d. Th. |
|---|---|
| Optische Reinheit: | 96,8 % ee |
| | $[\alpha]_D^{20}$ = +24,2° (c = 5 in $CHCl_3$) |

Beispiel 8

Entsprechend Beispiel 1 wurde eine 0,25 molare 3-Methylmercaptopropionaldehydlösung bei pH = 3,3 und T = 5°C umgesetzt. Die Reaktion war nach 90 Minuten beendet.

| Chemische Ausbeute: | 85,5 % d. Th. |
|---|---|
| Optische Reinheit: | 97,3 % ee |
| | $[\alpha]_D^{20}$ = +41.0° (c = 5 in $CHCl_3$) |

Beispiel 9

Entsprechend Beispiel 1 wurde eine 0,25 molare Hydrozimtaldehydlösung bei pH = 4,0 und T = 8°C umgesetzt. Die Reaktion war nach 80 Minuten beendet.

| Chemische Ausbeute: | 93,8 % d. Th. |
|---|---|
| Optische Reinheit: | 95,1 % ee |
| | $[\alpha]_D^{20}$ = -6,2° (c = 5 in $CHCl_3$) |

5

### Beispiel 10

Entsprechend Beispiel 1 wurde eine 0,25 molare Zimtaldehydlösung bei pH = 4,3 und T = 8°C umgesetzt. Die Reaktion war nach 90 Minuten beendet.

| Chemische Ausbeute: | 94 % d. Th. |
|---|---|
| Optische Reinheit: | 94,7 % ee |
| | $[\alpha]_D^{20}$ = +23,75° (c = 5 in CHCl$_3$) |

### Beispiel 11

Entsprechend Beispiel 1 wurde eine 0,25 molare Pivalinaldehydlösung bei pH = 3,3 und T = 6°C umgesetzt. Die Reaktion war nach 90 Minuten beendet.

| Chemische Ausbeute: | 81,4 % d. Th. |
|---|---|
| Optische Reinheit: | 85 % ee |
| | $[\alpha]_D^{20}$ = +13,2° (c = 5 in CHCl$_3$) |

### Beispiel 12

Entsprechend Beispiel 1 wurde eine 0,2 molare Butyraldehydlösung bei pH = 3,5 und T = 7°C umgesetzt. Die Reaktion war nach 2 Stunden beendet.

| Chemische Ausbeute: | 83 % d. Th. |
|---|---|
| Optische Reinheit: | 96,4 % ee |
| | $[\alpha]_D^{20}$ = +13,6° (c = 5 in CHCl$_3$) |

### Beispiel 13

Entsprechend Beispiel 1 wurde eine 0,2 molare Crotonaldehydlösung bei pH = 3,3 und T = 5°C umgesetzt. Die Reaktion war nach 2 Stunden beendet.

| Chemische Ausbeute: | 82 % d. Th. |
|---|---|
| Optische Reinheit: | 97,5 % ee |
| | $[\alpha]_D^{10}$ = +25,2° (c = 5 in CHCl$_3$) |

### Beispiel 14:

52 mg (0,5 mmol) para-Hydroxy-benzaldehyd wurden in 9,4 ml 50 mM Citratpuffer von pH 3,75 gelöst und auf 20°C temperiert. Hierzu wurden 500 $\mu$l (S)-Oxynitrilase-Lösung und 800 $\mu$l 4,2 M wäßrige HCN-Lösung hinzugegeben. Die eingesetzte (S)-Oxynitrilase-Lsg. hatte eine Aktivität von 84 U/ml, wobei 1 U die Bildung von einem $\mu$mol/min para-Hydroxymandelsäurenitril bei 20°C und pH 3,75 katalysiert.

Die Reaktion wurde polarimetrisch ($\lambda$ = 578 nm) verfolgt. Nach 15 bis 30 Min. stellte sich ein konstanter Drehwert ein, und die Reaktion war beendet. Danach wurde das Reaktionsgemisch viermal mit 10 ml Diethylether extrahiert. Die vereinigten organischen Phasen wuren über Natriumsulfat getrocknet und das Lösungsmittel am Rotationsverdampfer abgezogen. Anschließend wurde der Rückstand dreimal mit je 10 ml Pentan gewaschen und das Produkt im Vakuum getrocknet.

| Chem. Ausbeute: | 64,8 mg (87 % d. Th.) |
|---|---|
| Optische Reinheit: | 99 % ee |

Die Bestimmung der optischen Reinheit erfolgte als N,O-Bis-(Pentafluorpropional)-2-amino-1-(p-hydroxyphenyl)-ethanol-Derivat des p-Hydroxymandelsäurenitrils kapillargaschromatographisch nach H. Frank et al. Die Derivatisierung wurde ebenfalls wie in Beispiel 1 angegeben durchgeführt.

Beispiel 15:

Entsprechend Beispiel 14 wurden 61 mg (0,5 mmol) meta-Hydroxy-benzaldehyd bei pH 3,25 mit 450 $\mu$l 4,2 M wäßriger HCN-Lösung umgesetzt. Nach 30 bis 40 Minuten war die Reaktion beendet. Die Isolierung des (S)-3-Hydroxymandelsäurenitrils erfolgte wie in Beispiel 14 beschrieben.

| Chem. Ausbeute: | 67 mg (90 % d. Th.) |
|---|---|
| Optische Reinheit: | 98 % ee |

Die Bestimmung der optischen Reinheit erfolge über das N,O-Bis-Pentafluorpropionyl-2-amino-1-(m-hydroxyphenyl)ethanol gemäß Beispiel 1.

Beispiel 16:

Entsprechend Beispiel 14 wurden 60 mg (0,5 mmol) meta-Methyl-benzaldehyd mit 475 $\mu$l 4,2 M wäßriger HCN-Lösung und 500 $\mu$l Oxynitrilase-Lösung bei pH 3,25 umgesetzt. Nach ca. 45 Min. war die Reaktion beendet. Die Aufarbeitung erfolgte analog Beispiel 1 unter Gewinnung von (S)-3-Methylmandelsäurenitril.

| Chem. Ausbeute: | 59 mg (80 % d. Th.) |
|---|---|
| Optische Reinheit: | 96 % ee |

Die Bestimmung der optischen Reinheit erfolgte über das N,O-Bis-Pentafluorpropionylesteramid des entsprechenden Aminoalkohols gemäß Beispiel 1.

Beispiel 17:

Entsprechend Beispiel 14 wurden 53 mg (0,5 mmol) Benzaldehyd mit 475 $\mu$l 4,2 M wäßriger HCN-Lösung und 1500 $\mu$l (S)-Oxynitrilaselösung bei pH 3,25 umgesetzt. Nach ca. 45 Min. war die Reaktion beendet. Die Aufarbeitung erfoglte analog zu Beispiel 1.

| Chem. Ausbeute: | 48 mg (80 % d. Th.) |
|---|---|
| Optische Reinheit: | 96 % ee |

Die Bestimmung der optischen Reinheit erfolgte über das N,O-Bis-Pentafluorpropionylesteramid gemäß Beispiel 1.

Beispiel 18:

Kontinuierliche Produktion von (S)-para-Hydroxymandelsäurenitril im Rührkesselreaktor mit immobilisierter (S)-Oxynitrilase

Eine Übertragung des Reaktionsprinzips auf die Bildung größerer Produktmengen in kontinuierlicher Arbeitsweise erfolgte mit an Eupergit ® C (Röhm , Darmstadt) immobilisierter (S)-Oxynitrilase.

In kontinuierlicher Arbeitsweise wurde in einem 10 ml Reaktor über 72 h mit einer Raumzeitausbeute von 57,9 g/(l • d) (S)-para-Hydroxymandelsäurenitril produziert.

| Charakteristische Reaktionsdaten: | | |
|---|---|---|
| para-Hydoxybenzaldehyd: | 21 mmol/l | |
| HCN: | 400 mmol/l | |
| Na-Citrat: | 45 mmol/l | ph 3,75 |
| Verweilzeit: | 3960 sec | |
| Katalysatorkonzentration: | 0,15 g/ml | |
| Laufzeit: | 72 h | |
| Enzymdesaktivierung: | ca. 3 %/d | |
| Reaktionsumsatz: | 85 % | |
| Enantiomerenüberschuß: | > 98 % | |
| Temperatur: | 20 °C | |

**Patentansprüche**

1. Verfahren zur Herstellung von optisch aktiven Cyanhydrinen durch enzymatische Umsetzung von Oxoverbindungen mit Blausäure in Gegenwart von Oxynitrilase,
   **dadurch gekennzeichnet,**
   daß man von aliphatischen, aromatischen oder heteroaromatischen Aldehyden oder Ketonen abgeleitete (R)-Cyanhydrine oder (S)-Cyanhydrine durch Umsetzung der entsprechenden Oxoverbindungen mit Blausäure in rein wäßrigem Milieu in Gegenwart von R-Oxynitrilase (4.1.2.10) bzw. Oxynitrilase (4.1.2.11) unter derart sauren Bedingungen und bei solchen Temperaturen herstellt, daß die chemische Konkurrenzreaktion und die Racemisierung gegenüber der enzymatischen Synthese vernachlässigbar sind.

2. Verfahren nach Anspruch 1,
   **dadurch gekennzeichnet,**
   daß man die Reaktion bei pH ≤ 4,5 durchführt.

3. Verfahren nach Anspruch 1 oder 2,
   **dadurch gekennzeichnet,**
   daß man eine Reaktionstemperatur von -5° bis +50°C wählt.

4. Verfahren nach einem der vorangehenden Ansprüche,
   **dadurch gekennzeichnet,**
   daß man die Reaktion kontinuierlich durchführt.

5. Verfahren nach Anspruch 4,
   **dadurch gekennzeichnet,**
   daß man die Reaktion in einem kontinuierlich betriebenen Rührkesselreaktor mit Enzym-Rückhaltung durchführt.

6. Verfahren nach einem der vorangehenden Ansprüche,
   **dadurch gekennzeichnet,**
   daß man die Umsetzung mit einem aromatischen Aldehyd durchführt.

**Claims**

1. Process for preparing optically active cyanohydrins by the enzymic reaction of oxo compounds with hydrocyanic acid in the presence of oxynitrilase, characterized in that (R)-cyanohydrins or (S)-cyanohydrins derived from aliphatic, aromatic or heteroaromatic aldehydes or ketones are prepared by reacting the corresponding oxo compounds with hydrocyanic acid in purely aqueous medium in the presence of R-oxynitrilase (4.1.2.10) or oxynitrilase (4.1.2.11) under such acidic conditions and at such temperatures that the competitive chemical reaction and the racemization are negligible as compared with the enzymic synthesis.

2. Process according to Claim 1, characterized in that the reaction is carried out at pH ≤ 4.5.

8

3. Process according to Claim 1 or 2, characterized in that a reaction temperature of from -5° to +50°C is selected.

4. Process according to one of the preceding claims, characterized in that the reaction is carried out continuously.

5. Process according to Claim 4, characterized in that the reaction is carried out in a continuously operated stirred-tank reactor using enzyme retention.

6. Process according to one of the preceding claims, characterized in that the reaction is carried out using an aromatic aldehyde.

**Revendications**

1. Procédé de préparation de cyanhydrines optiquement actives par réaction enzymatique de composés oxo avec l'acide cyanhydrique en présence d'oxynitrilase, caractérisé en ce qu'on prépare des (R)-cyanhydrines ou des (S)-cyanhydrines dérivées d'aldéhydes ou de cétones aliphatiques, aromatiques ou hétéroaromatiques, par mise en réaction des composés oxo correspondants avec de l'acide cyanhydrique dans un milieu purement aqueux, en présence de R-oxynitrilase (4.1.2.10) ou d'oxynitrilase (4.1.2.11), dans des conditions acides et à des températures telles que la réaction chimique compétitive et la racémisation sont négligeables par rapport à la synthèse enzymatique.

2. Procédé selon la revendication 1, caractérisé en ce qu'on effectue la réaction à pH ≤ 4,5.

3. Procédé selon la revendication 1 ou 2, caractérisé en ce qu'on choisit une température de réaction de -5°C à +50°C.

4. Procédé selon l'une des revendications précédentes, caractérisé en ce qu'on effectue la réaction en continu.

5. Procédé selon la revendication 4, caractérisé en ce qu'on effectue la réaction dans un réacteur à agitation fonctionnant en continu, avec retenus de l'enzyme.

6. Procédé selon l'une des revendications précédentes, caractérisé en ce qu'on effectue la réaction avec un aldéhyde aromatique.